# EUROPEAN PATENT APPLICATION

(11) **EP 4 062 935 A1**
(43) Date of publication of application: **28.09.2022**
(21) Application number: 20891254.3
(22) Date of filing: 17.11.2020
(51) Int. Cl.: A61K 39/395, A61K 47/00, A61K 51/04, A61P 35/00, B82Y 5/00

(54) **THERANOSTIC SYSTEM FOR DIRECTED DIFFUSION OF THERAPEUTIC AND IMAGING AGENTS TO CANCER CELLS**

(30) Priority: 20.11.2019 ES 201931015
(71) Applicant: Universitat Politècnica de València, 46022 Valencia (ES); Consejo Superior de Investigaciones Científicas (CSIC), 28006 Madrid (ES)
(72) Inventor: BOTELLA ASUNCIÓN, Pablo, 46022 Valencia (ES); RIVERO BUCETA, Eva María, 46022 Valencia (ES)
(74) Representative: Pons
(86) International application number: PCT/ES2020/070714
(87) International publication number: WO 2021/099662

(57) **Abstract**

The invention relates to a multifunctional system stable in a physiological medium, which includes in the same platform an anti-carcinogenic molecule, an imaging agent and a directing molecule that interacts specifically with cancer-cell membrane receptors, the system allowing pathological tissue imaging and pharmacological action to be carried out jointly with high specificity. The intratumoral administration of the system facilitates selective diffusion to cancer cells and minimises the disadvantages of chemotherapy.

## Description

The invention relates to a multifunctional system stable in a physiological medium, defined as theranostic, which includes in the same platform an anti-carcinogenic molecule, an imaging agent and a directing molecule that interacts specifically with cancer-cell membrane receptors, the system allowing pathological tissue imaging and pharmacological action to be carried out jointly with high specificity. Likewise, the intratumour administration of said theranostic system facilitates selective diffusion to cancer cells and minimises the disadvantages of chemotherapy.

### BACKGROUND OF THE INVENTION

Parenteral administration of antitumour agents can lead to serious toxicity problems, which drastically limits the dosage of the therapeutic agent and thus the efficacy of the treatment. In this sense, including the biologically active molecule on a pharmaceutical carrier that facilitates selective diffusion to cancer cells minimises the disadvantages of chemotherapy. This is especially evident when two premises are met:
i) direct intratumour administration, instead of the parenteral route;
ii) including a directing molecule in the pharmaceutical carrier that guarantees the specific interaction with tumour-cell membrane receptors.

In this context, the nanoparticles of certain porous materials have enormous potential as pharmaceutical carriers capable of incorporating numerous functionalities on the surface thereof and inside the pores, as therapeutic agents, fluorochromes, paramagnetic elements, radionuclei and directing molecules, as well as protective substances that guarantee the stability in biological fluids and minimal or no immunogenicity. In this way, the same system, defined as theranostic, would allow pathological tissue imaging and pharmacological action to be carried out in a simple bolus administration.

In the particular case of prostate cancer (PCa), the evolution of the tumour takes place over a prolonged period, wherein during the T phases the tumour only affects the gland, and during the N phase the tumour affects the gland and spreads to nearby lymph nodes. Therefore, it is feasible to carry out treatment with chemotherapeutic agents using intratumour administration. Said treatment, however, would not be recommended once the M phase (metastasis) of PCa has been reached. Likewise, given that certain PCa cell lines develop specific membrane receptors, such as the FOLH1 receptor, also called prostate-specific membrane antigen (PSMA), including molecules of an antibody on the surface of nanoparticulate carriers that specifically interact with said receptors will allow very high levels of efficacy and selectivity to be reached in the treatment of the pathology, as well as an image resolution at the cellular level using the appropriate instrumentation.

Therefore, there is an urgent need to develop new pharmaceutical carriers based on nanoparticles made of porous materials carrying antitumour molecules and imaging agents, capable of carrying out specific antigen-antibody interactions with cancer cells, for intratumour administration thereof.

Patent WO2014145606A1 describes silica nanoparticles with theranostic usefulness comprising a fluorescent compound, polyethylene glycol (PEG), a monoclonal antibody directed against prostatic PSMA and a therapeutic agent such as, for example, DTX. The nanoparticles may also comprise a contrasting agent consisting of a radionucleus selected from: 89Zr, 64Cu, 68Ga, 86Y, 1241 and 177Lu.

Moreover, E. Rivero-Buceta et al. (ACS Omega 2019, 4, 1281-1291), develop MCM-41-type porous silica nanoparticles functionalised with DTX and an anti-FOLH1 monoclonal antibody (clone C803N) directed against FOLH1 receptors for the treatment of prostate cancer, obtaining high selectivity by the LNCaP cell line, on which a much higher cytotoxic activity is obtained than that of free DTX.

Likewise, P. K. B. Nagesh et al. (Colloids and Surfaces B: Biointerfaces 2016, 144, 8-20) synthesise SPION-type paramagnetic nanoparticles comprising an iron oxide core and layers of β-cydodextrin and poloxamer 407 (Pluronic^{®} F-127). These nanoparticles are loaded with DTX and also include the anti-FOLH1 monoclonal antibody (clone J591), directed against FOLH1 receptors. The system is highly effective against prostate cancer and provides a high degree of internalisation of DTX in C4-2 cells, as well as in tumours containing this cell line.

Furthermore, the document by A. S. Oliveira (Development of new nanocarriers for controlled release of Olanzapine and Camptothecin. 2018. 141f. Tese, Universidade Federal do Rio Grande do Norte, Natal, 2018) describes the synthesis of a carrier for the administration of camptothecin based on a COF-5-type covalent crganic framework with primary amine groups to which 20-O-camptothecin succinate is covalently bound, since it is a 100 % organic and biodegradable material, which would solve the problems of accumulation in the body of nanoparticle residues that can be produced with inorganic materials, such as silica and iron oxide. In addition, due to the high specific surface thereof, COF is capable of including a high number of molecules of the bioactive agent, facilitating the administration thereof at low dosages. However, the inclusion of amine groups (and therefore the bonding of camptothecin) in the framework is limited by the electronic repulsion they generate inside the same, which destabilises the hexagonal lamellar structure (2D) of the material. This notably reduces the crystallinity, as well as the stability in the physiological medium, causing the rapid dissolution of the particles and releasing the pharmacological load in an uncontrolled manner. In this sense, this system was not very effective against cultures of the HeLa cancer-cell line.

In the present invention, the synthesis of nanoparticles of stable covalent organic frameworks in physiological medium is detailed, solving the problem of partial or total dissolution observed in the previously described boronic covalent frameworks, which allows the use thereof for cancer diagnosis and therapy, including in the same platform an anticancer molecule, an imaging agent and a directing molecule that specifically interacts with cancer-cell membrane receptors. In this way, a multifunctional system defined as theranostic is obtained, which allows the acquisition of images of pathological tissues and pharmacological action to be carried out with great specificity. Likewise, the intratumour administration of said theranostic system facilitates selective diffusion to cancer cells and minimises the disadvantages of chemotherapy.

### DESCRIPTION OF THE INVENTION

The present invention relates to a theranostic system stable in a physiological medium that specifically interacts with FOLH1 receptors of cells or tissues, and that comprises nanoparticles of a porous material characterised in that said porous material has a crystalline structure that corresponds to a covalent organic framework (COF) to which the following elements are bound through terminal amino groups present in the structure thereof:
- at least one therapeutic molecule,
- an imaging agent, and
- an anti-FOLH1 monoclonal antibody that interacts specifically with FOLH1 receptors,
wherein the nanoparticles have a diameter greater than 20 nm (for example, determined by dynamic light scattering, DLS), and more preferably a diameter between 20 and 500 nm.

In a particular embodiment, the nanoparticles have a mean pore diameter of between 2 and 10 nm.

In the present invention, covalent organic framework is understood as a new class of porous organic materials that have crystalline structures in two (2D) or three (3D) dimensions due to the covalent bonds that bind the different organic units or monomers that form said frameworks.

In a particular embodiment, the organic monomer that makes up the covalent organic framework is selected from boronate, boroxine, borosilicate, borazine, imide, amide, hydrazone, amine, dioxine, triazine, imine, β-ketoenamine, azine, thiazole, oxazole, azodioxide, and a viologen;

In another additional particular embodiment, the porous material of the nanoparticles has a crystalline structure that corresponds to an covalent organic framework selected from among the following materials: COF-5 *(covalent organic framework 5),* COF-10 (*covalent organic framework 10),* COF-42 (*covalent organic framework 42*), COF-43 (*covalent organic framework 43),* COF-66 (*covalent organic framework 66*), COF-117 (*covalent organic framework 117),* COF-118 (*covalent organic framework 118*), COF-336 *(covalent organic framework 336),* COF-367 (*covalent organic framework 367*), TPB-DMTP-COF (*triphenylbenzene-dimethoxyterephaldehyde-covalent organic framework*), HHTP-DPB-COF (*2,3,6,7,10,11-hexahydroxytriphenylene- 4,4'-diphenylbutadiynebis(boronic acid)-covalent organic framework*)*,* TP-COF (*triphenylene covalent organic framework*), COF-S-SH (*covalent organic framework sulfur derivative*), NiPc-COF (*nickel phthalocyanine-covalent organic framework*), ZnPc-PPE-COF (*zinc phthalocyanine-phenylbis(phenylethynyl)-covalent organic framework),* [C₆₀]-ZnPc-COF (*[C₆₀]- zinc phthalocyanine-covalent organic framework),* MC-COF-NiPc-E₁E₇ *(multiple component-covalent organic framework-nickel phthalocyanine-1,4-benzene diboronic acid-p-phenylenediamine*), CuPc-FPBA-DETHz (*2,3,9,10,16,17,23,24-octahydroxyphthalocyaninato-4-formylphenylboronic acid-2,5-diethoxyterephthalohydrazide*), TPE-Ph-COF (*tetraphenylethene-phenyl-covalent organic framework*), Py-Azine-COF (*pyrene-azine-covalent organic framework*), TTF-Py-COF (*tetrathiafulvalene-pyrene-covalent organic framework*), HBC-COF (*hexabenzocoronene covalent organic framework*), ICOF-2 (*ionic covalent organic framework 2),* Star-COF (*star-covalent organic framework*), CCOF-2 (*chiral covalent organic framework 2),* y 3D-Py-COF (*3D-pyrene-covalent organic framework*).

In the present invention, amino group is understood as a covalent bonding point of organic ligands carrying a bioactive molecule, therapeutic molecule being understood as an imaging agent or a monoclonal antibody. For its part, the methoxy group reduces the electronic repulsion in the structure of the covalent organic framework through a resonance effect of the aromatic ring, substantially improving the crystallinity of the material and the stability of the nanoparticles in a physiological medium, delaying or preventing the dissolution thereof.

In another particular embodiment, the therapeutic molecule of the theranostic system is selected from at least one small molecule, a macromolecule, an antibody, a peptide, a protein, an enzyme, a nucleic acid, and an organic polymer. In the field of the present invention, macromolecule is understood as any molecule of biological or synthetic origin the molecular weight of which is greater than 3,000 Da. Likewise, small molecule is understood as any molecule of biological or synthetic origin the molecular weight of which is less than 3,000 Da. In addition, the system of the present invention may contain one or more types of therapeutic molecules.

In an additional particular embodiment, the therapeutic molecule is bound to the nanoparticles of the theranostic system through covalent or non-covalent bonding.

In an additional preferred particular embodiment, the therapeutic molecule is an antitumour agent, selected from camptothecin, doxorubicin, docetaxel, paclitaxel, and cabazitaxel, or a combination thereof.

In an even more preferred additional particular embodiment, the therapeutic molecule is bound to the nanoparticles of the theranostic system through an organic ligand or linker that facilitates the covalent bonding thereof to a terminal amino group through a bio-hydrolysable bond selected from an ester, amide, carbamate or carbonate. Said organic ligand is an unbranched hydrocarbon chain of length *n,* at the ends of which two carboxyl groups are arranged, and wherein n relates to the number of carbons in said chain, which varies between 1 and 4. Even more preferably, the therapeutic molecule is docetaxel (DTX), and the organic ligand or linker is succinic acid, which is bound at one end to DTX in the 2'-OH position by means of an ester bond (Suc-DTX) and by the opposite end to a terminal amino group by means of an amide bond. In this way, the release of the therapeutic molecule in the intracellular environment and the cytotoxic activity thereof depends on the action of cytoplasmic esterases.

Among the advantages of the theranostic system of the invention is the improvement in the bioavailability of the therapeutic molecule, while the interactions thereof with other therapeutic molecules and with blood plasma components are reduced. Likewise, the immune response is reduced or eliminated, it is protected from the metabolism, the release kinetics are modulated, the plasma residence time and half-life are increased, the toxicity and side effects associated with its administration are reduced, the efficiency is increased, its metabolism is normalised in subjects of different species and ethnic groups and/or races, and the directed diffusion to specific tissues and/or cells is promoted.

In the field of the present invention, the concentration of the therapeutic molecule can range between 0.1 and 40 % by weight, more preferably between 3 and 15 % by weight when the therapeutic molecule is a small molecule or a macromolecule.

In the present invention, an imaging agent is understood as a substance that, once introduced into the body, is capable of emitting a signal in order to highlight the tissue or organ to be examined. Once inside the organism, a piece of equipment for detecting said agent will identify the signals emitted and create images with great precision, leading to more detailed information being obtained.

In a preferred embodiment, the imaging agent of the theranostic system is selected from a fluorochrome, a paramagnetic element, and a radionucleus.

In an additional particular embodiment, the imaging agent is bound to the nanoparticles of the theranostic system by covalent bonding.

In an additional more preferred particular embodiment, the imaging agent is bound to the nanoparticles of the theranostic system through an organic ligand or linker that facilitates the covalent bonding thereof to a terminal amino group through an amide or carbamate bond.

In another additional particular embodiment, the imaging agent of the theranostic system is a fluorochrome that is selected from among fluorescein, rhodamine, coumarin, and cyanine, or a combination thereof. In the field of the present invention, the concentration of the fluorochrome can range between 0.01 and 10% by weight, more preferably between 0.5 and 2% by weight.

In another additional particular embodiment, the imaging agent of the theranostic system is a paramagnetic element that is selected from Gd (III), Fe (III), Mn (II), and ¹⁹F, or a combination thereof. In the field of the present invention, the concentration of the paramagnetic element can range from 0.01 to 10% by weight.

In another additional particular embodiment, the imaging agent of the theranostic system is a radionucleus selected from a β⁺ emitter such as ¹⁸F, ⁶⁴Cu, ⁶⁸Ga, ⁸⁹Zr, ⁹⁹Tc, ¹⁷⁷Lu, an Auger electron emitter, such as ¹¹¹In, ¹²⁵I, an α emitter such as ²¹¹At, ²¹²Bi, ²¹³Bi, ²²⁵Ac, and ²²⁷Th, or a combination thereof. In the field of the present invention, the concentration of the radionucleus can range from 0.0001 to 1% by weight.

In another particular embodiment, the anti-FOLH1 monoclonal antibody is selected from an anti-FOLH1 monoclonal antibody of synthetic, semi-synthetic, or natural origin.

In an additional particular embodiment, the anti-FOLH1 monoclonal antibody is bound to the nanoparticles of the theranostic system by covalent bonding.

For purposes of the present invention, the term "antibody" relates to immunoglobulin molecules, or to immunologically active portions of immunoglobulin molecules, i.e., molecules that contain an antigen-binding site that is specifically bound (immunoreacts) to the FOLH1 protein (PROTEIN DATA BANK ID FOLH1: 1Z8L). Examples of immunologically active portions of immunoglobulin molecules include F(ab) and F(ab')2 fragments, which can be generated by treating the antibody with an enzyme such as pepsin, or in a recombinant manner.

For the purposes of the present invention, all access numbers to the PROTEIN DATA BANK database are up to date as of November 2019.

In a preferred embodiment, the antibody of the invention may be polyclonal (typically includes different antibodies directed against different determinants or epitopes) or monoclonal (directed against a single determinant on the antigen). The term "monoclonal antibody" relates to a population of antibody molecules that contain only one species of antigen-binding site capable of immunoreacting with a particular epitope of the antigen.

The monoclonal antibody may be altered biochemically, by means of genetic manipulation, or may be synthetic, the antibody possibly lacking in whole or in part portions that are not required for antigen recognition. In a preferred embodiment, the antibody, as described for use thereof in the present invention, is preferably a monoclonal antibody.

The term "antigen" for purposes of the present invention relates to a predetermined region to which the antibody is able to bond selectively. The antigen may be a polypeptide, carbohydrate, nucleic acid, lipid, hapten, or other natural or synthetic molecule. Preferably, the antigen is a polypeptide, more preferably, the antigen is the FOLH1 protein (PROTEIN DATA BANK ID FOLH1: 1Z8L).

In another preferred embodiment of the monoclonal antibody of the system of the invention, it is characterised in that it can be a recombinant, humanised, chimeric, synthetic antibody or a combination of any of the above. A "recombinant antibody" (rAC) is an antibody that has been produced in a host cell transformed or transfected with a polynucleotide encoding the FOLH1 peptide, with a nucleic acid encoding the antibody of the invention, or that produces the antibody that specifically binds to the FOLH1 protein (PROTEIN DATA BANK ID FOLH1: 1Z8L), or the FOLH1 protein, as a result of homologous recombination. A "chimeric antibody" is an antibody wherein a region of the heavy and/or light chain is identical or homologous to corresponding sequences in antibodies from a particular species or belonging to a particular class or subclass of antibodies, while the remaining chain(s) is(are) identical to, or homologous to, the corresponding sequences in antibodies derived from other species or belonging to another class or subclass of antibodies, as well as to fragments of said antibodies, such that they exhibit the desired biological activity. A "synthetic antibody" is an antibody that is generated using recombinant DNA technology. It should also be considered that the term is understood as an antibody that has been generated by means of the synthesis of a DNA molecule encoding the antibody and that said DNA molecule expresses an antibody protein, or an antibody-specific amino acid sequence, wherein the DNA or amino acid sequence has been obtained using DNA or synthetic amino acid sequencing technology that is available and well known in the art.

In a more preferred embodiment, any anti-FOLH1 monoclonal antibody known in the art can be used in the present invention. More particularly, the anti-FOLH1 monoclonal antibody (PROTEIN DATA BANK ID FOLH1: 1Z8L) is selected from the list consisting of: J591 (The Prostate 2014, 74, 1674-1690), J415 (The Prostate 2014, 74, 1674-1690), D2B (J. Nucl. Med. 2014, 55, 995-1001), 107-1A4 (NOVUS BIOLOGICALS), GCP-05 (INVITROGEN), 2G7 (The Prostate 2014, 74, 768-780), YPSMA-1 (ABCAM), YPSMA-2 (ANOGEN), GCP-02 (Neuroscience 2007, 144, 1361-1372), GCP-04 (NOVUS BIOLOGICALS), 3E6 (DAKO), 24.4E6 (The Prostate 2014, 74, 768-780), C803N (CREATIVE DIAGNOSTICS) and 7E11-C5.3 (J. Nucl. Med. 2003, 44, 610-617).

In an additional more preferred particular embodiment, the anti-FOLH1 monoclonal antibody is bound to the nanoparticles of the theranostic system through an organic ligand that facilitates the covalent bonding thereof (linker) to a terminal amino group by means of an amide or carbamate bond. Said organic ligand is an unbranched hydrocarbon chain of length n, which contains a carboxyl group at one end and an amino group at the other, and wherein *n* relates to the number of carbons in said chain, which varies between 7 and 13. In a preferred embodiment, the organic ligand or linker is selected from 10-aminododecanoic acid, 9-aminononanoic acid, 11-chloro-11-oxoundecanoic acid and 11-aminoundecanoic acid; and even more preferably, the organic ligand or linker is 11-aminoundecanoic acid, which is bound by covalent bond to a terminal amino group on the surface of the nanoparticle, allowing the directing molecule (antibody) to be separated from FOLH1 receptors (PROTEIN DATA BANK ID FOLH1: 1Z8L) of the therapeutic molecule, in order to avoid the possible steric hindrance associated with the proximity of two bulky molecules. In the field of the present invention, the amount of antibody linked to the nanoparticles can range between 0.0008 and 0.16 µmol/g.

In another particular embodiment, the theranostic system of the present invention comprises nanoparticles the surface of which is covered with terminal amino groups, and a molecule of a poly(ethylene glycol) (PEG) covalently bound to a terminal amino group of the nanoparticle. The PEG molecule increases the solubility and stability of the material in an aqueous medium, allowing the preparation of stable colloids while reducing the interactions of the theranostic system with blood plasma components, increasing plasma residence time, reducing or eliminating the immune response, reduces toxicity and increases efficacy, and promotes directed diffusion to specific tissues and/or cells.

In an additional particular embodiment, the PEG molecule has a molecular weight of between 200 and 5,000 Da. In the field of the present invention, the concentration of PEG can range between 0.05 and 10 % by weight, more preferably between 0.5 and 3% by weight.

A second aspect of the present invention is the use of the theranostic system defined above in a biotechnological application and which, in the field of the present invention, relates to the diagnosis and/or treatment of those types of cancer wherein malignant cells develop FOLH1-type membrane receptors (prostate, breast, lung, colorectal and kidney, among others), and which comprises putting one or more *"in vitro"* cells in contact with the system of the invention.

In a preferred embodiment, said biotechnological application is the diagnosis and/or treatment of prostate, breast, lung, colorectal and kidney cancer; and more preferably the diagnosis and/or treatment of prostate cancer. Therefore, another aspect of the invention is the use of the theranostic system of the invention in the diagnosis and/or treatment of prostate adenocarcinoma.

It has been proven that most prostate adenocarcinoma cell lines develop FOLH1 membrane specific receptors (PROTEIN DATA BANK ID FOLH1: 1Z8L) also called prostate-specific membrane antigen (PSMA), for example, but not limiting the field of the present invention: RWPE-1, PIN Cells, RWPE-2, LNCaP, LAPC-4, LAPC-9, VCaP, MDA PCa 2a/2b, LuCaP, C4-2, C4-2B, 22Rv1, ARCaP. Therefore, the incorporation on the surface of the nanoparticles of an antibody that selectively interacts with said receptors (anti-FOLH1), favours the selective diffusion of the theranostic system inside the tumour cells.

A third aspect of the present invention consists of the administration of the theranostic system in stable colloid form by means of parenteral or intratumour administration. In a preferred way, the administration is carried out by means of intratumour injection on the prostate gland. Said route of administration substantially limits the diffusion of the theranostic system to the rest of the body.

Both the presence of the anti-FOLH1 antibody (PROTEIN DATA BANK ID FOLH1: 1Z8L) in the theranostic system, such as the intratumour administration protocol, minimise the activity of the system on other organs and tissues other than the prostate, avoiding unwanted effects, while locally increasing the concentration of both the therapeutic molecule and the imaging agent.

From the point of view of chemotherapy alone, the activity and selectivity of the theranostic system administered intratumourly are at a maximum when the cancer is in the T and N phases of the disease, and its efficacy decreases in the M phase.

In the field of the present invention, the equivalent concentration of the therapeutic molecule intratumourly administered to the patient with the theranostic system ranges between 0.0001 mg/(kg h) and 10 mg/(kg h), for 1 to 2000 h. Equivalent concentration is understood as the required amount of nanoparticles to which the therapeutic molecule is bound, which must be administered in order to incorporate the established or reference concentration of the drug or active ingredient.

In the field of the present invention, the concentration of nanoparticles of the theranostic system administered to the patient in the prostate tissue varies between 1 and 1000 µg/ml, and preferably between 5 and 200 µg/ml.

In the field of the present invention, the equivalent concentration of therapeutic molecule in the cell medium required to cause a cytotoxic action ranges between 0.005 µg/ml and 200 µg/ml.

Throughout the description and in the claims, the word "comprises" and its variants are not intended to exclude other technical features, additives, components or steps. For those skilled in the art, other objects, advantages and features of the invention may be deduced from both the description and the practical use of the invention. The examples that are provided by way of illustration, and are not meant to limit the present invention.

### BRIEF DESCRIPTION OF THE FIGURES

**FIG.** 1 is a schematic illustration of the functionalisation sequence of nanoparticles of the COF-5 porous material with terminal amino and methoxy groups by covalent anchoring of the therapeutic molecule Suc-DTX, the imaging agent [¹⁸F]FDG, the spacer chain 11-aminoundecanoic acid, and the anti-FOLH1 antibody (PROTEIN DATA BANK ID FOLH1: 1Z8L).
**FIG.** 2 is a schematic illustration of the functionalisation sequence of nanoparticles of the COF-5 porous material with terminal amino and methoxy groups by covalent anchoring of the B rhodamine imaging agent, the spacer chain 11-aminoundecanoic acid, and the anti-FOLH1 antibody (PROTEIN DATA BANK ID FOLH1: 1Z8L).
**FIG.** 3 Image obtained in the transmission electron microscope (TEM) showing the morphology of nanoparticles of the COF-25 material of Example 3.
**FIG.** 4 Representation of the powder diffraction patterns obtained for nanoparticles of the COF-0 (COF-5) and COF-25 materials obtained in Example 3, and presented in Table 1.
**FIG.** 5 DTX release curve from the theranostic system of Example 4 in phosphate buffered saline (PBS, 1x, pH 7.4). Experimental conditions: T = 37 °C; stirring = 1500 rpm; concentration = 5 mg/ml.

### EXAMPLES

The invention is illustrated below by means of tests carried out by the inventors which reveal the effectiveness of the product of the invention.

### Materials

### EXAMPLE 1: Suc-DTX synthesis

200 mg of DTX (0.24 mmol, I), 50 mg of succinic anhydride (0.500 mmol), and 1 mg of 4-(dimethylamino)pyridine (DMAP, 0.008 mmol) are introduced in a 50 ml flask. An anhydrous mixture of 12 ml (11:1) of dichloromethane (DCM) and dimethylformamide (DMF) is then injected. The solution is kept under stirring for 24 h at room temperature and under an inert atmosphere. After this time, the solvent is evaporated under vacuum (50 torr), a white solid being obtained, which is redissolved in 30 ml of ethyl acetate. The organic phase is then washed with aqueous hydrochloric acid solution (1%, w/v) (3 x 30 ml), and afterwards with ultrapure water (3 x 30 ml). The organic phase is separated, dried over anhydrous magnesium sulphate, and the solvent is evaporated under vacuum (50 torr), a white solid being obtained. Said white residue is dissolved in the minimum amount of solvent and purified by means of a chromatographic column, using DCM and methanol (100%→96:4%, v/v) as mobile phase.

The fraction containing the desired product, the solvent is evaporated under vacuum (50 torr), and 168 mg (75%) of a white solid (Suc-DTX, **II**) are obtained.

¹H-RMN (300 MHz, dmso-d₆) 0.97 (s, 6H); 1.38 (s, 9H); 1.51 (s, 3H); 1.68 (s, 3H); 2.23 (s, 3H); 2.51 (2H, below solvent signal); 2.61 (d, 2H, J=6.3 Hz); 3.61 (m, 2H); 4.03 (m, 3H); 4.44 (s, 1H); 5.00 (m, 5H); 5.39 (d, 1H); 5.77 (m, 1H); 7.19 (t, 1H); 7.39 (m, 4H); 7.69 (m, 3H); 7.87 (d, 1H); 7.98 (d, 2H); 12.83 (sₐ, 1H); ¹³C-RMN (75 MHz, dmso-d₆) 9.74; 13.64; 20.72; 22.43; 26.40; 28.47; 28.62; 28.72; 30.73; 34.90; 36.56; 42.82; 45.94; 51.32; 55.08; 56.92; 70.70; 71.04; 73.69; 74.76; 75.33; 76.77; 78,46; 80,25; 83,71; 127,42; 128,01; 128,50; 128,64; 129,52; 130,00; 133,34; 135,96; 136,75; 137,39; 155,16; 162,27; 165,24; 168,82; 169,50; 171,45; 172,55; 172,97; 209,28. Q-TOF MS (ESI, m/z) [M-Na]⁻ calculated for C₄₇H₅₇NO₁₇Na, 930.3524; found 930.3502.

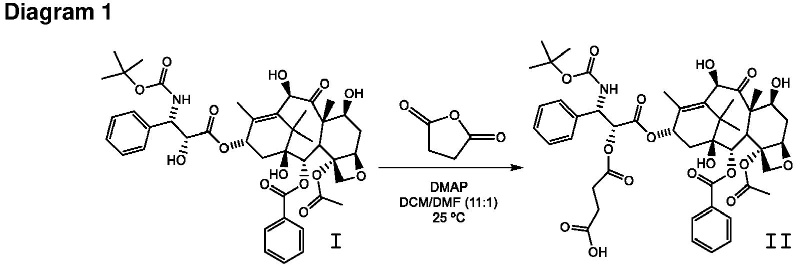

### EXAMPLE 2: 2-amino-5-methoxy-benzene-1,4-diboronic acid synthesis

2.96 g (11.13 mmol) of 1,4-dibromo-5-methoxybenzene are dissolved in anhydrous tetrahydrofuran (100 ml) and the temperature of the mixture is lowered to -78 °C. Next, 14.8 ml of n-BuLi (23.65 mmol, 1.6 M in hexane) are added drop by drop for 30 minutes. Once the n-BuLi has been added, stirring is maintained for 5 hours under refrigeration. After this time, 5.4 ml of trimethyl borate (48.30 mmol) are slowly added and magnetic stirring of the mixture is maintained at -78 °C. It is left stirring overnight, allowing it to reach room temperature. After this time, acetic acid is added and the mixture is stirred for 2 hours at room temperature. The solvent is evaporated under vacuum, the suspension is filtered and washed with water. The compound is recrystallised in a water/acetonitrile mixture (100 ml, 50:50 v/v), filtered and the solid obtained is dried under vacuum (8 torr) for 24 hours. 1.02 g (47%) of 5-methoxy-1,4-benzenediboronic acid (MDMB) are obtained.

30 ml of 90% nitric acid are cooled at 0 °C for 15 minutes. Next, 100 mg of urea (1.67 mmol) are added and the temperature of the mixture is lowered to -10 °C. Subsequently, 5.91 g (30.1 mmol) of 2-methoxy-1,4-benzenediboronic acid (MBDB) is added in small portions with strong stirring for 1 hour. After completing the addition of the MBDB, the magnetic stirring of the mixture is maintained at -10 °C for an additional 15 minutes. After this time, the dark red solution is poured onto an ice bath containing a small amount of water. The suspension is filtered, washed with water, and the solid obtained is dried under vacuum (8 torr) for 24 hours. 4.36 g (60%) of a yellow solid (2-nitro-5-methoxy-1,4-benzenediboronic acid (NO₂-MBDB)) are obtained.

¹H-RMN (300 MHz, dmso-d₆) 4.13 (s, 3H); 7.53 (s, 1H); 8.54 (s, 1H). ¹³C-RMN (75 MHz, dmso-d6) 56.2; 115.28; 124.80; 125.46; 135.40; 139.34; 168.48. Q-TOF MS (ESI, m/z) [MH]⁻ calculated for C₇H₁₀B₂NO₇, 241.05; found 240.75.

In a second reaction step, to a solution of 570 mg of NO₂-MBDB in a mixture of 10 ml of methanol and 0.2 ml of 36.5 % hydrochloric acid, 150 mg of palladium supported on carbon (Pd/C, 10 % wt Pd) are added. The treatment is carried out in a hydrogenation reactor (H₂, 10 bar) at room temperature and with stirring for 1 hour. After this time, the suspension is filtered over Celita^{®} to separate the palladium supported on carbon. The fraction containing the desired product, the solvent is evaporated under vacuum (50 torr), and 580 mg (99.4%) of a white solid are obtained (2-amino-5-methoxy-1,4-benzenediboronic acid (NMBB)).

¹H-RMN (300 MHz, dmso-d₆) 3.83 (s, 3H); 7.46 (s, 1H); 7.61 (s, 1H); ¹³C-RMN (75 MHz, dmso-de) 56.24; 115.11; 116.35; 125.30; 128.03; 128.60; 143.60; 152.38. Q-TOF MS (ESI, m/z) [MH]⁻ calculated for C₇H₁₂B₂NO₅, 211.07; found 210.76.

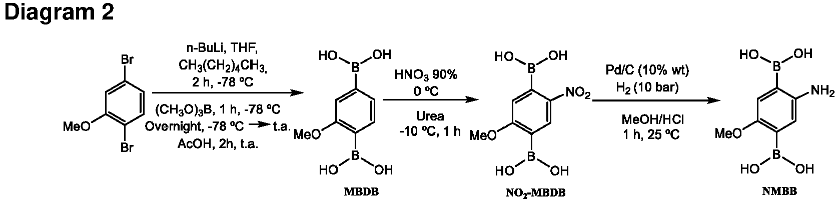

### EXAMPLE 3: Preparation of nanoparticles of the COF-25 porous material with terminal methoxy and amino groups.

For the synthesis of COF-5 derivatives with terminal amino groups, the sequential replacement of MBDB by NMBB was carried out during the condensation of the covalent organic framework. The material obtained is designated by the nomenclature CF-x, wherein x represents the degree of replacement: percentage of NMBB over the total bi-coordinated ligand (MBDB + NMBB).

Initially, 243 mg of HHTP (0.749 mmol) are added to a solution of MBDB (221 mg, 1.128 mmol) and CH₃OH (0.47 ml, 11.7 mmol) in 75 ml of an anhydrous 1,4-dioxane:mesitylene (4:1 v/v) mixture. The resulting suspension is subjected to sonication for 1 minute and filtered (0.45 µm PTFE) to introduce it into a Schlenk tube. Next, 187 ml of anhydrous acetonitrile and a further 112 ml of the anhydrous 1,4-dioxane/mesitylene (4:1, v/v) mixture are injected to obtain nanoparticles with a 2 mM concentration of HHTP in a 50% (v/v) acetonitrile. The mixture is purged with N₂ and 2 cycles of N ₂/vacuum are carried out to remove any traces of moisture. The reaction mixture is left under magnetic stirring at 90 °C for 20 hours. After this time, the suspension is filtered, washed with anhydrous toluene, and the solid obtained is dried under vacuum (8 torr) for 24 hours. 206 mg of nanoparticles of the COF-25 porous material are collected.

The sequential replacement of MBDB by NMBB led to obtaining the different COF-x materials, the composition of which is summarised in Table 1.

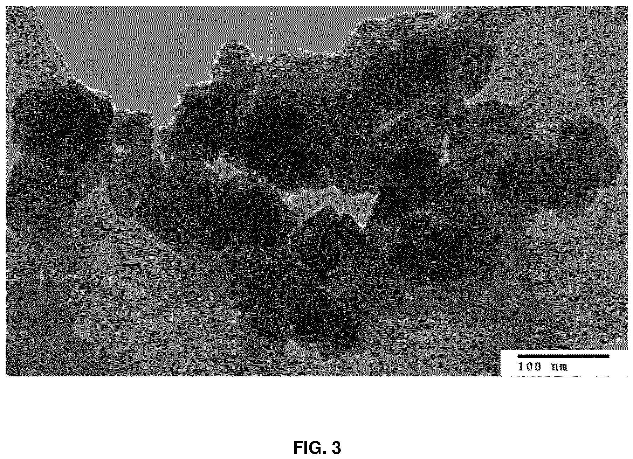

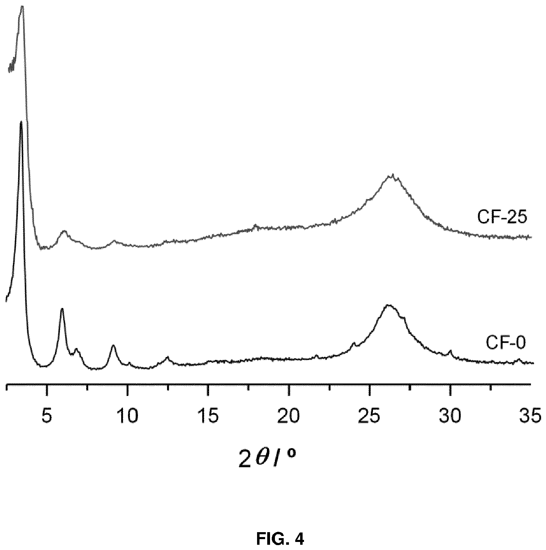

Figure 3 shows an image of the nanoparticles of the COF-25 material obtained in this example. Likewise, Figure 4 shows the X-ray diffractograms of the COF-0 materials (it does not incorporate terminal amino groups) and COF-25 materials obtained in this example.

**Table 1 Initial composition used for the preparation of nanoparticles of the COF-5 material and COF-x derivatives stable in physiological environment.**

| COF-x | x [%] | Molar ratio | Composition of the synthesis gel | | | | | |
|---|---|---|---|---|---|---|---|---|
| | | (MBDB: HHTP:NMBB) | HHTP (mg) | HHTP (mmol) | MBDB (mg) | MBDB (mmol) | NMBB (mg) | NMBB (mmol) |
| **COF-0** | 0 | 3.00 : 2 : 0.00 | 243 | 0.75 | 187 | 0.96 | --- | --- |
| **COF-25** | 25 | 2.53 : 2 : 0.86 | 243 | 0.75 | 186 | 0.95 | 68 | 0.32 |
| **COF-40** | 40 | 1.91 : 2 :1.29 | 243 | 0.75 | 140 | 0.72 | 102 | 0.48 |
| **COF-60** | 57 | 1.54 : 2 : 2.06 | 243 | 0.75 | 113 | 0.58 | 163 | 0.77 |
| **COF-70** | 71 | 1.27 : 2 : 3.11 | 243 | 0.75 | 93 | 0.48 | 246 | 1.17 |
| **COF-80** | 78 | 1.04 : 2 : 3.71 | 243 | 0.75 | 76 | 0.39 | 293 | 1.39 |

### EXAMPLE 4: Preparation of nanoparticles of the COF-25 porous material with terminal methoxy and amino groups on which Suc-DTX is bound by means of covalent bond.

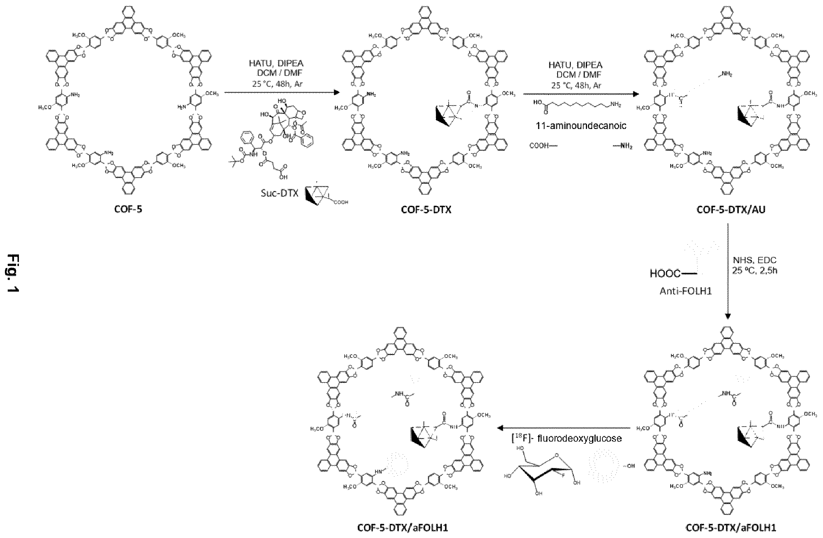

200 mg of the COF-25 porous material obtained in Example 3 are dried (100 °C, vacuum, 24 hours). Next, they are suspended in 20 ml of anhydrous DCM, 200 mg of the prodrug Suc-DTX (0.22 mmol, prepared according to Example 1) are added, 84 mg 1-oxide-3-(bis(dimethylamino)methylene)-3H-[1,2,3]triazolo[4,5-bipiri-dine hexafluorophosphate (V) (HATU, 0.22 mmol) and 115 µl N,N'-diisopropylethylamine (DIPEA, 0.66 mmol). The reaction mixture is left stirring at room temperature under an argon atmosphere for 48 h. After this time, the suspension is filtered, washed with anhydrous DCM and the grey solid obtained is dried under vacuum (8 torr) for 24 hours. 224 mg of nanoparticles of the CF-25 porous material are collected the surface of which is covered with terminal amine groups to which Suc-DTX molecules are bound by amide bond (COF-25-DTX, Figure 1).

### EXAMPLE 5: Preparation of nanoparticles of the COF-25 porous material with terminal methoxy groups and terminal amino groups on which Suc-DTX and a PEG molecule are bound by covalent bond

200 mg of the COF-25-DTX porous material obtained in Example 4 are dried (80 °C, vacuum, 24 hours), and suspended in 30 ml of anhydrous DCM, and 250 µl of N,N'-diisopropylethylamine (DIPEA, 0.46 mmol) are added under argon. Next, 150 mg of 2,5,8,11-tetraoxatetradecane-14-oic acid succinimidyl ester (PEG₃) is added and the mixture is stirred at room temperature for 16 h. After removing the solvent on a rotary evaporator, the solid is re-dispersed by stirring in 100 ml of ethanol. This suspension is filtered and washed with ethanol. Finally, the solid is lyophilised (-55 °C, 16 h). 268 mg of nanoparticles of the COF-25-DTX@PEG porous material are collected the surface of which is covered with terminal amine groups to which Suc-DTX and PEG ₃ molecules are bound by amide bond.

### EXAMPLE 6: Preparation of nanoparticles of the COF-25 porous material with terminal methoxy groups and terminal amino groups on which Suc-DTX, the acid-11-aminoundecanoic linker and the anti-FOLH1 directing molecule (clone C803N, Creative Diagnostics; PROTEIN DATA BANK ID FOLH1: 1Z8L) are bound by covalent bond.

100 mg of COF-25-DTX nanoparticles obtained according to Example 4 are dried (60 °C, vacuum, 24 hours), and suspended in 10 ml of anhydrous DCM. Next, 61 mg of 11-aminoundecanoic acid (AU, 0.30 mmol), 114 mg of HATU (0.30 mmol) and 157 µl of DIPEA (0.90 mmol) are added, and it is left stirring at room temperature and under argon atmosphere for 48 hours. After this time, the suspension is filtered, washed with anhydrous DCM, and the solid obtained is dried under vacuum (8 torr) for 24 hours. 115 mg of nanoparticles of COF-25 porous material are obtained the surface of which is covered with terminal amine groups to which Suc-DTX and 11-aminoundecanoic acid molecules are bound, respectively, by amide bond (COF-25-DTX/AU, Figure 1).

10 µl of anti-FOLH1 antibody (clone C803N, Creative Diagnostics, 5 mg/ml) are suspended in 100 µl of commercial 2-(N-morpholino) ethanesulphonic acid monohydrate buffer (MES, 0.1 M, pH 6.0). Next, 100 µl of a solution of N-(3-dimethylaminopropyl)-N'-ethylcarbodiimide (EDC, 8 mg/ml) and 100 µl of a solution of N-hydroxysuccinimide (NHS, 20 mg/mL) are added, and left stirring at room temperature for 30 minutes. After this time, 1 mg of COF-25-DTX/AU nanoparticles obtained according to the previous step are dispersed in 1 ml of PBS (1x, pH 8.5) and added to the previously prepared antibody solution. It is left stirring for 2 h at room temperature. At the end of the reaction period, the sample is centrifuged (16100 g), the residue is washed with PBS (1x, pH 7.4), lyophilised at -55 °C for 16 hours and stored at 4 °C until use thereof (COF-25-DTX/aFOLH1, Figure 1).

The amount of antibody bound by amide bond to the terminal amino group of the AU spacer chain is determined by the Bradford method. In this assay, the amount of protein (antibody) not conjugated to the particles is quantified by visible spectrophotometry (□= 595 nm) through the binding that is established between a dye (Coomassie blue G-250) and the protein under study.

### EXAMPLE 7: Preparation of nanoparticles of the COF-5 porous material with terminal methoxy groups and terminal amino groups on which Suc-DTX, the imaging agent [¹⁸F]FDG, the 11-aminoundecanoic acid linker, and the anti-FOLH1 directing molecule (clone C803N, Creative Diagnostics; (PROTEIN DATA BANK ID FOLH1: 1Z8L) are bound by covalent bond.

A freshly obtained aliquot of [¹⁸F]-fluorodeoxyglucose [¹⁸F]FDG (-3 mCi) is dried at 110 °C and vacuum. 1 ml of a suspension in sterile COF-25-DTX/aFOLH1 nanoparticles (1 mg/ml) Milli Q^{®} water obtained in Example 6 is added on the dry [¹⁸F]FDG. The resulting suspension is stirred at room temperature for 15 min in a sterile environment. After this time, it is centrifuged (16100 g) and washed several times with sterile Milli Q^{®} water. The final mixture contains 1 mg/ml of COF-25-DTX/aFOLH1/[¹⁸F] in sterile Milli Q^{®} water and is ready for intratumour or parenteral administration.

### EXAMPLE 8: Preparation of nanoparticles of the COF-25 porous material with terminal methoxy groups and terminal amino groups on which the imaging agent rhodamine B is bound by covalent bond.

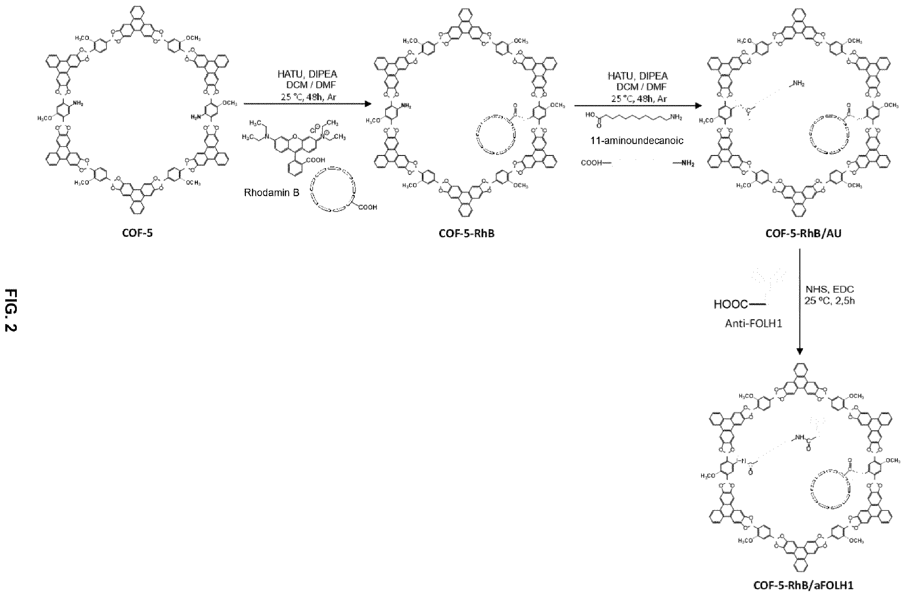

200 mg of the COF-25 porous material obtained in Example 3 are dried (100 °C, vacuum, 24 hours). It is subsequently suspended in 10 ml of anhydrous DCM. Next, 3.0 mg of rhodamine B (0.006 mmol), 11 mg of HATU (0.030 mmol) and 16 µl of DIPEA (0.090 mmol) are added, and it is left stirring at room temperature and under an argon atmosphere for 48 hours. After this time, the suspension is filtered, washed with anhydrous DCM, and the solid obtained is dried under vacuum (8 torr) for 24 hours. 233 mg of nanoparticles of the COF-25 porous material are collected the surface of which is covered with terminal amine groups to which rhodamine B molecules are bound by amide bond (COF-25-RhB, Figure 2).

### EXAMPLE 9: Preparation of nanoparticles of the COF-25 porous material with terminal methoxy groups and terminal amino groups on which the imaging agent rhodamine B, the 11-aminoundecanoic acid linker, and the anti-FOLH1 directing molecule (clone C803N, Creative Diagnostics; (PROTEIN DATA BANK ID FOLH1: 1Z8L) are bound by covalent bond.

100 mg of COF-25-RhB nanoparticles obtained according to Example 8 are dried (60 °C, vacuum, 24 hours), and suspended in 10 ml of anhydrous DCM. Next, 61 mg of 11-aminoundecanoic acid (AU, 0.30 mmol), 114 mg of HATU (0.30 mmol) and 157 µl of DIPEA (0.90 mmol) are added, and it is left stirring at room temperature and under argon atmosphere for 48 hours. After this time, the suspension is filtered, washed with anhydrous DCM, and the solid obtained is dried under vacuum (8 torr) for 24 hours. 112 mg of nanoparticles of the COF-25 porous material are obtained the surface of which is covered with terminal amine groups to which rhodamine B and 11-aminoundecanoic acid molecules are bound, respectively, by amide bond (COF-25-RhB/AU, Figure 2).

10 µl of anti-FOLH1 antibody (clone C803N, Creative Diagnostics, 5 mg/ml) is suspended in 100 µl MES (0.1 M, pH 6.0). Next, 100 µl of a solution of EDC (8 mg/ml) and 100 µl of a solution of NHS (20 mg/ml) are added, and left stirring at room temperature for 30 minutes. After this time, 1 mg of COF-25-RhB/AU nanoparticles obtained according to the previous step are dispersed in 1 ml of PBS (1x, pH 8.5) and added to the previously prepared antibody solution. It is left stirring for 2 h at room temperature. At the end of the reaction period, the sample is centrifuged (16100 g), the residue is washed with PBS (1x, pH 7.4), lyophilised at -55 °C for 16 hours and stored at 4 °C until use (COF-25-RhB/aFOLH1, Figure 2).

### EXAMPLE 10: DTX release in phosphate buffered saline.

A 5 mg/ml suspension of nanoparticles obtained according to Example 4 is prepared in 1 ml of PBS in a 2 ml eppendorf tube and incubated at 37 °C for the different times established. The samples incubated at the different times are centrifuged, the residue is washed with methanol (2 x 1 ml) and the supernatant is lyophilised at -55 °C for 24 h. The residue obtained is reconstituted with 1 ml of a methanol solution. 5 µl of said solution are injected into an HPLC equipment. The DTX peak area is integrated and compared to a calibration curve. All measurements were made in triplicate, calculating the mean ± standard deviation (SD) in each case.

It is verified that the material presents great stability in PBS, with a release of around 10% of the total load in the first hour of incubation, characterised as a mixture of free DTX and Suc-DTX (approximately 20% of the total). This initial release is related to prodrug molecules adsorbed inside the pores. Subsequently, the release of DTX is low until at least 6 h of exposure (15% of the total load).

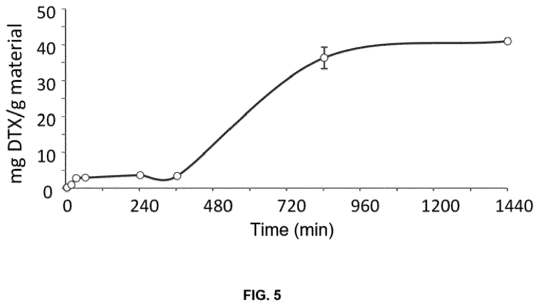

Figure 5 shows the release curve in PBS of DTX from the COF-25-DTX theranostic system.

### EXAMPLE 11: Determination of the IC₅₀ by means of MTT assay

The *in vitro* cytotoxic activity of the theranostic system was determined by the 3-[4,5-dimethylthiazol-2-yl]-2,5-diphenyltetrazolium (MTT) bromide test. In these assays, the proliferation of living cells is determined via mitochondrial dehydrogenase activity: viable mitochondria reduce MTT by generating MTT-formazan crystals that are quantifiable by visible spectrophotometry.

Two types of human prostate adenocarcinoma cell lines were used for these assays: LNCaP (with membrane receptors FOLH1, PROTEIN DATA BANK ID FOLH1: 1Z8L) and PC3 (without FOLH1 receptors).

The cells are seeded in 96-well plates at a rate of 10,000 cells/well in both cases, 24 h before incorporating the nanoparticles. Aliquots of 3 mg ml-¹ of DTX in DMSO are prepared and kept at -20 °C. When required, an aliquot is thawed, and working aliquots (100 µg/ml by dilution in enriched RPMI medium) are prepared. Alternatively, aliquots of 100 µg/mL DTX are prepared in enriched RPMI medium and kept at -20°C until required.

A suspension of nanoparticles obtained according to Example 4 is prepared in RPMI medium enriched with 10% foetal bovine serum and 1% penicillin/streptomycin at 37 °C, in a humid air (95%) and CO₂ (5%) atmosphere, with an equivalent concentration of 100 µg/ml of DTX and treated with ultrasound to improve the dispersion of the nanoparticles. The suspension is divided into aliquots that are kept at -20 °C until required.

Alternatively, a suspension of nanoparticles obtained according to Example 6 is prepared in RPMI medium enriched with 10% foetal bovine serum and 1% penicillin/streptomycin at 37 °C, in a humid air (95%) and CO₂ (5%) atmosphere, with an equivalent concentration of 100 µg/ml DTX and treated with ultrasound under identical conditions.

The cells are treated with DTX or with nanoparticles obtained according to Example 4, in a range of DTX doses between 0.005 µg/ml and 100 µg/ml for 72 hours. Alternatively, the cells are treated with nanoparticles obtained according to Example 6 for 72 hours. At the end of the incubation period, 10 µl (5 mg/ml) of the MTT solution are added to each well and 4 hours later the formazan crystals are dissolved with isopropanol, and the absorbance at 570 nm is measured. The percentage of cell survival relative to untreated cells is determined and the values of *IC*₅₀ from the curve DTX concentration-(%) live cells are calculated. The values of *IC*₅₀ are listed in Table 2.

**Table 2. Values of IC₅₀ (mean ± SD, in µg/ml) for free DTX, COF-25-DTX from Example 4 and COF-25-DTX/aFOLH1 from Example 6 in PC3 and LNCaP cells (n = number of experiments).**

| Cell line | DTX | COF-25-DTX | COF-25-DTX/aFOLH1 | n |
|---|---|---|---|---|
| LNCaP | 0.80 ± 0.20 | 0.14 ± 0.02 | 0.02 ± 0.01 | 3 |
| PC3 | - | - | - | 3 |

## Claims

1. A theranostic system comprising nanoparticles of a porous material **characterised in that** said porous material has a crystalline structure that corresponds to a covalent organic framework to which the following elements are bound through terminal amino groups present in the structure thereof:
- at least one therapeutic molecule,
- an imaging agent, and
- an anti-FOLH1 monoclonal antibody that interacts specifically with FOLH1 receptors,
wherein the nanoparticles have a diameter greater than 20 nm.

2. The theranostic system according to claim 1, **characterised in that** the nanoparticles have a diameter comprised between 20 and 500 nm.

3. The theranostic system according to any of the preceding claims, **characterised in that** the nanoparticles have a pore diameter of between 2 and 10 nm.

4. The theranostic system according to any of the preceding claims, **characterised in that** the covalent organic framework is made up of organic monomers selected from boronate, boroxine, borosilicate, borazine, imide, amide, hydrazone, amine, dioxin, triazine, imine, β-ketoenamine, azine, thiazole, oxazole, azodioxide, and a viologen.

5. The theranostic system according to any of the preceding claims, **characterised in that** the porous material of the nanoparticles has a crystalline structure that is selected from COF-5, COF-10, COF-42, COF-43, COF-66, COF-117, COF-118, COF-336, COF-367, TPB-DMTP-COF, HHTP-DPB-COF, TP-COF, COF-S-SH, NiPc-COF, ZnPC-PPE-COF, [C₆₀]-ZnPc-COF, MC-COF-NiPc-E₁E₇, CuPc-FPBA-DETHz, TPE-Ph-COF, Py-Azine-COF, TTF-Py-COF, HBC-COF, ICOF-2, Star-COF, CCOF-2 and 3D-Py-COF.

6. The theranostic system according to any of the preceding claims, **characterised in that** the structure also has terminal methoxy groups.

7. The theranostic system according to any of the preceding claims, **characterised in that** the therapeutic molecule is selected from a small molecule of biological or synthetic origin the molecular weight of which is less than 3,000 Da, a macromolecule, an antibody, a peptide, a protein, an enzyme, a nucleic acid or an organic polymer.

8. The theranostic system according to claim 7, **characterised in that** the small therapeutic molecule is an antitumour agent, selected from camptothecin, doxorubicin, docetaxel, paclitaxel, and cabazitaxel, or a combination thereof.

9. The theranostic system according to claim 8, **characterised in that** the concentration of the therapeutic molecule can range between 0.1 and 40% by weight.

10. The theranostic system according to any of claims 8-9, **characterised in that** the therapeutic molecule is bound to the nanoparticles by covalent bond.

11. The theranostic system according to claim 10, **characterised in that** the therapeutic molecule is bound to the nanoparticles through a linker that facilitates the covalent bond thereof to the terminal amino group through a bio-hydrolysable bond selected from an ester, amide, carbamate or carbonate.

12. The theranostic system according to claim 11, **characterised in that** the therapeutic molecule is docetaxel (DTX), and the linker is succinic acid.

13. The theranostic system according to any of the preceding claims, **characterised in that** the imaging agent is selected from a fluorochrome, a paramagnetic element, and a radionucleus.

14. The theranostic system according to claim 13, **characterised in that** the imaging agent is bound to the nanoparticles by covalent bond.

15. The theranostic system according to claim 14, **characterised in that** the imaging agent is bound to the nanoparticles through a linker that facilitates the covalent bond thereof to a terminal amino group through an amide or carbamate bond.

16. The theranostic system according to claim 13, **characterised in that** the fluorochrome is selected from fluorescein, rhodamine, coumarin, and cyanine, or a combination thereof.

17. The theranostic system according to claim 16, **characterised in that** the concentration of the fluorochrome can range between 0.01 and 10 % by weight.

18. The theranostic system according to claim 13, **characterised in that** the paramagnetic element is selected from Gd (III), Fe (III), Mn (II), and ¹⁹F, or a combination thereof.

19. The theranostic system according to claim 18, **characterised in that** the concentration of the paramagnetic element can range between 0.01 and 10 % by weight.

20. The theranostic system according to claim 13, **characterised in that** the radionucleus is selected from a β⁺ emitter such as ¹⁸F, ⁶⁴Cu, ⁶⁸Ga, ⁸⁹Zr, ⁹⁹Tc, ¹⁷⁷Lu, an Auger electron emitter, such as ¹¹¹In, ¹²⁵I, an α emitter such as ²¹¹At, ²¹²Bi, ²¹³Bi, ²²⁵Ac, and ²²⁷Th or a combination thereof.

21. The theranostic system according to claim 20, **characterised in that** the concentration of the radionucleus can range between 0.0001 and 1 % by weight.

22. The theranostic system according to any of the preceding claims, **characterised in that** the anti-FOLH1 monoclonal antibody is selected from an anti-FOLH1 monoclonal antibody of synthetic, semi-synthetic or natural origin.

23. The theranostic system according to any of the preceding claims, **characterised in that** the anti-FOLH1 monoclonal antibody is selected from the list consisting of: J591, J415, D2B, 107-1A4, GCP-05, 2G7, YPSMA-1, YPSMA-2, GCP-02, GCP-04, 3E6, 24.4E6, clone C803N and 7E11-C5.3.

24. The theranostic system according to claim 22, **characterised in that** the anti-FOLH1 monoclonal antibody is bound to the nanoparticles by covalent bond.

25. The theranostic system according to claim 24, **characterised in that** the anti-FOLH1 monoclonal antibody is bound to the nanoparticles through a linker by means of an amide or carbamate bond.

26. The theranostic system according to claim 25, **characterised in that** the linker is selected from 10-aminododecanoic acid, 9-aminononanoic acid, 11-chloro-11-oxoundecanoic acid and 11-aminoundecanoic acid.

27. The theranostic system according to claim 26, **characterised in that** the linker is 11-aminoundecanoic acid.

28. The theranostic system according to any of claims 22 to 27, **characterised in that** the amount of antibody linked to the nanoparticles can range between 0.0008 and 0.16 µmol/g.

29. The theranostic system according to any of the preceding claims, **characterised in that** it further comprises a PEG molecule that is bound to the nanoparticle by covalent bond to a terminal amino group.

30. The theranostic system according to claim 29, **characterised in that** the PEG molecule has a molecular weight between 200 and 5,000 Da.

31. The theranostic system according to any of claims 29 to 30, **characterised in that** the concentration of PEG can range between 0.05 and 10% by weight.

32. A theranostic system according to any of the preceding claims for use as a medicament.

33. The theranostic system according to any of claims 1 to 31, for use in the treatment of prostate, breast, lung, colorectal, and kidney cancer.

34. The theranostic system for use according to claim 33, wherein the prostate cancer is a prostate adenocarcinoma.

35. The theranostic system for use according to claim 33 or 34, **characterised in that** the route of administration is selected from intratumoral or parenteral.

36. The theranostic system for use according to claim 35, **characterised in that** the route of administration is performed by intratumour injection on the prostate gland.

37. The theranostic system for use according to claim 35, **characterised in that** the equivalent concentration of the therapeutic molecule administered intratumourly to the patient ranges between 0.0001 mg/(kg h) and 10 mg/(kg h), for 1 to 2000 h.

38. The theranostic system for use according to claim 34, **characterised in that** the concentration of nanoparticles administered to the patient in the prostate tissue varies between 1 and 1000 µg/ml, and preferably between 5 and 200 µg/ml.
